# EUROPÄISCHE PATENTANMELDUNG

(11) **EP 0 638 328 A1**
(43) Veröffentlichungstag der Anmeldung: **15.02.1995**
(21) Anmeldenummer: 94109387.4
(22) Anmeldetag: 17.06.1994
(51) Int. Cl.: F16K 15/14, A61M 39/00

(54) **Rückschlagventil**

(30) Priorität: 14.07.1993 DE 9310673 U
(71) Anmelder: B. BRAUN MELSUNGEN AG, D-34212 Melsungen (DE)
(72) Erfinder: Fuchs, Jürgen, D-34308 Bad Emstal (DE); Lesemann, Egon, D-34212 Melsungen (DE)
(74) Vertreter: Selting, Günther, Dipl.-Ing.

(57) **Zusammenfassung**

Die Erfindung besteht in einem Rückschlagventil
mit einem Gehäuse (10) mit axialem Durchlaß (13) und einem Ventilsitz, mit dem eine Ventilscheibe (33) aus elastischem Material zusammenwirkt, die innerhalb eines Dichtringes (31) angeordnet und über eine örtliche Anschlußstelle (32) mit dem Dichtring (31) verbunden ist, wobei der Dichtring (31) zwischen einer oberen und einer unteren Ringfläche (19, 26) des Gehäuses (10), die den Ventilsitz (20) konzentrisch umgeben, eingespannt ist und wobei das Gehäuse (10) in eine Flüssigkeitsleitung eines Infusions- oder Transfusionsgerätes eingesetzt ist und der Durchlaß (13) Flüssigkeit führt.

Ein solches Rückschlagventil ist u.a. besonders geeignet, Rückströmungen von Flüssigkeiten bei Infusionen, insbesondere Mehrfachinfusionen, zu Infusionslösungsbehältnissen hin zu verhindern. Es wird größtmögliche Sicherheit bei der Verwendung von Infusionspumpen erreicht.

## Beschreibung

Die Erfindung bezieht sich auf ein Rückschlagventil, bestehend aus einem Gehäuse mit axialem Durchlaß und einer Ventilscheibe aus elastischem Material, die zur Absperrung des Durchlasses mit einem Ventilsitz zusammenwirkt.

Rückschlagventile haben die Aufgabe, Rückströmungen von fließfähigen Medien in Leitungen zu verhindern. Bekannt sind Kugelventile (US-A-45 76 594), Scheibenventile (U-79 17 008) und sogenannte Entenschnabelventile (U-70 16 939). Diese Ventile sind nicht nur teilweise umständlich aufgebaut, sondern sie haben auch einige konstruktionsbedingte Nachteile. Kugelventile schließen nur dann dicht, wenn sie in einem kegelförmigen Sitz sitzen. Dann besteht aber die Gefahr eines Festsetzens der Kugel. Scheibenventile sind im allgemeinen lageabhängig und außerdem arbeiten sie nur bei bestimmten Druckunterschieden bzw. bei bestimmten Strömungsgeschwindigkeiten einwandfrei, d. h. für undefinierte, sehr geringe Drücke sind sie ungeeignet. Bei Entenschnabelventilen ergeben sich durch einbaubedingte Vorspannungen in dem geschlitzten Becherkörper Undichtigkeiten an den Schlitzlippen, so daß die Ventile unzuverlässig sind.

Ferner ist aus EP-A-0247 824 ein Ventil für ein medizinisches Infusionssystem bekannt, bei dem in einstückiger Ausbildung eine dicke Ventilscheibe, eine diese mit Abstand umgebende Dichtwulst und eine dünne Trägerbahn vorgesehen sind, die die Ventilscheibe mit der Dichtwulst verbindet. In der Trägerbahn sind umfangsmäßige Löcher ausgebildet, die als Fluiddurchlaß dienen, wenn die Ventilscheibe von einem Ventilsitz abgehoben ist. Im Originalzustand liegt die Trägerbahn auslenkungsfrei in der Ebene der Ventilscheibe. Im eingebauten Zustand wird sie von der gegen den Ventilsitz anliegenden Ventilscheibe in Querrichtung gespannt. Diese Dauerbeanspruchung der Trägerbahn führt im Laufe der Zeit zu Ermüdungserscheinungen und es besteht die Gefahr, daß nach längerer Lagerung des Ventils seine Dichtfunktion nicht mehr zuverlässig gewährleistet ist.

Zum Stande der Technik zählt die nicht vorveröffentlichte EP-Patentanmeldung 0582 038, die eine Schutzvorrichtung für einen Belüftungskanal einer Tropfkammer beschreibt, die mit einem Rückschlagventil und einem Bakterienfilter versehen ist. Das Rückschlagventil weist eine in einem waagerechten Gehäuse senkrecht ausgerichtete Dichtmembran auf, die über einen Radialabschnitt innerhalb eines sie mit Abstand umgebenden Dichtringes angeordnet ist, der zwischen zwei Flächen eingespannt ist, die einen waagerechten Lufteinlaß umgeben. Die Dichtmembran wird durch Luftdruck betätigt.

Der Erfindung liegt die Aufgabe zugrunde, Rückströmungen von Flüssigkeiten bei Transfusionen oder Mehrfachinfusionen zu den Flüssigkeitsbehältnissen hin zuverlässig zu verhindern.

Diese Aufgabe wird durch die Merkmale des Anspruches 1 gelöst.

Ein derartiges Rückschlagventil ist mit besonderem Vorteil Bestandteil einer Leitungsanordnung zur Übertragung von Flüssigkeiten, wenn mit der Leitungsanordnung gleichzeitig sowohl Flüssigkeitslösungen unter Schwerkraft als auch solche, die durch (Infusions)-Pumpen gefördert werden, einem Empfänger zugeführt werden. Die Kombination von (peristaltischen) Infusionspumpen oder Infusionsspritzenpumpen mit Infusionsgeräten zur Schwerkraftinfusion bei einer zeitgleichen Parallelinfusion (Mehrfachinfusion) ist allgemein üblich, jedoch bisher nicht sicher genug.

Das erfindungsgemäße Rückschlagventil ist besonders geeignet, Rückströmungen von Flüssigkeiten bei Infusionen, insbesondere Mehrfachinfusionen, zu den Infusionslösungsbehältnissen hin zu verhindern. Es verbessert die Funktion der Infusionsgeräte mit oder ohne Belüftung durch sofortiges Schließen des Leitungsdurchlasses bei Auftreten eines Rückstaus der Infusionslösung, gleichgültig, aus welchem Grunde dieser eintritt. Es wird größtmögliche Sicherheit bei der Verwendung von Infusionspumpen erreicht.

Im Normalfall fließt die Infusionslösung in den Durchlaß des im wesentlichen senkrecht ausgerichteten Gehäuses und drückt bei einer Wassersäule von ca. 10 cm die Ventilscheibe von dem Ventilsitz weg, so daß der Durchlaß geöffnet wird. Sind in einem Mehrfachinfusionsleitungssystem sowohl Schwerkraftleitung(en) als auch energetisch unterstützte Pumpenleitung(en) kombiniert, kann durch eine Leitungsstenose, z. B. eine im Patienten liegende, verschlossene Kanüle, die Infusionslösung aus der Pumpenleitung(en) in Richtung der Schwerkraftleitung(en) und das damit verbundene Infusionslösungsbehältnis(se) fließen. Eine nicht kontrollierte Patientenversorgung ist damit vorgegeben.

Sobald der Fließdruck der von der Infusionspumpe kommenden Infusionslösung höher ist als der Fließdruck der von einem Infusionsbehältnis über ein Schwerkraftinfusionsgerät ankommenden, lediglich der Schwerkraft unterworfenen Infusionslösung, wird die Ventilscheibe gegen den Ventilsitz gedrückt und schließt den Durchlaß dicht ab. Die Funktionsfähigkeit des Ventils und die unidirektionale Abdichtfähigkeit des Ventilkörpers müssen vorzugsweise und vor allem auch bei einem von der Pumpe erzeugten Schließfluß im Bereich von nur 1 ml/h geförderter Infusionslösung(en) gegeben sein. Es wird so zuverlässig verhindert, daß die mit der Infusionspumpe(n) geförderte Infusionsflüssigkeit(en) in den Leitungsweg(e) des Schwerkraftinfusionsgerätes(e) gelangt. Das sofortige Ansprechen der Ventilscheibe auf den Infusionspumpendruck ist insbesondere auf die Vorspannung der nur an einer örtlichen Verbindungsstelle an dem Dichtring befestigten Ventilscheibe zurückzuführen. Unter der Bezeichnung "Dichtring" wird ein Ventilkörperring mit permanenter Dichtfunktion verstanden.

Vorzugsweise sind die zusammenpassenden oberen und unteren Ringflächen des Gehäuses nach außen abfallend abgeschrägt und es liegt die Stirnfläche des ringförmigen Ventilsitzes in der Ebene der tiefsten Stelle der oberen Ringfläche des Gehäuses. Damit liegt der Dichtring auf einer flachen Kegelstumpffläche, während die Ventilscheibe gegen die gerade Ebene des ringförmigen Ventilsitzes anliegt. Auf diese Weise ergibt sich die Vorspannung der Ventilscheibe in Richtung des Ventilsitzes, d. h. in Schließrichtung. Es ist vorteilhaft, wenn die Abschrägung der oberen und unteren Ringflächen des Gehäuses zu der die Längsachse des Gehäuses rechtwinklig kreuzenden Ebene unter einem Winkel > 0 ° und < 90 ° verläuft. Diese Fixierschräge ist für die Vorspannung der Ventilscheibe besonders günstig. Die Befestigung des Dichtringes zwischen den beiden Ringflächen des Gehäuses kann durch Klebung und/oder Klemmung erfolgen.

In der Ebene unterhalb der unteren Ringfläche kann mindestens eine axiale Nase angeordnet sein, die als Anschlag für die Ventilscheibe dient und die verhindert, daß die Ventilscheibe in Strömungsrichtung der Flüssigkeit soweit in den Durchlaß hineingedrängt wird, daß sie ihn verschließt. Zweckmäßigerweise ist ein Kranz von Nasen vorgesehen, die den Durchlaß umgeben.

Die Ventilscheibe und der Dichtring sind bevorzugt aus Flachmaterial einstückig ausgebildet und die Anschlußstelle ist als radialer Steg gestaltet. Das Flachmaterial der Ventilscheibe und des Dichtringes kann Naturkautschuk, Silikonkautschuk oder ein thermoplastisches Elastomer sein. Vorzugsweise ist die Ventilscheibe siliconiert.

Die Ventilfunktion ist steuerbar über die Scheibendicke, Shore-Härte des Materials, Ausführung der Kante des ringförmigen Ventilsitzes und der Vorspannung. Hierdurch ist es möglich, den Öffnungs- und Schließdruck funktionsentsprechend zu beeinflussen.

Zwischen dem Dichtring und der Ventilscheibe ist ein der Länge des Stegs entsprechender umfangsmäßiger Zwischenraum vorhanden. Die Länge, Breite und wesentlich die Dicke des Stegs sind mitbestimmend für die Spannkraft, mit der die Ventilscheibe gegen den ringförmigen Ventilsitz anliegt. Die beiden Seitenränder des Stegs können einwärts gebogen sein; sie können auch gegen den Dichtring konvergieren. Die Auswahl der Stegform richtet sich nach der Größe der Ventilscheibe, die in Anpassung an den Durchmesser des Durchlasses des Gehäuses gewählt ist.

Für einen Einbau in Infusions- oder Transfusionsleitungen ist es günstig, daß das rohrförmige Gehäuse zweiteilig mit einem Schlauchansatz mit dem Ventilsitz und der oberen Ringfläche und aus einem Konnektor-Ansatz mit der unteren Ringfläche gebildet ist, die einen Spalt zur klemmenden Aufnahme des Dichtringes definieren. Diese Ausbildung des Gehäuses erlaubt eine Kombination mit Leitungsanschlüssen in üblicher Weise.

In der Zeichnung ist ein Ausführungsbeispiel der Erfindung schematisch dargestellt. Es zeigt:

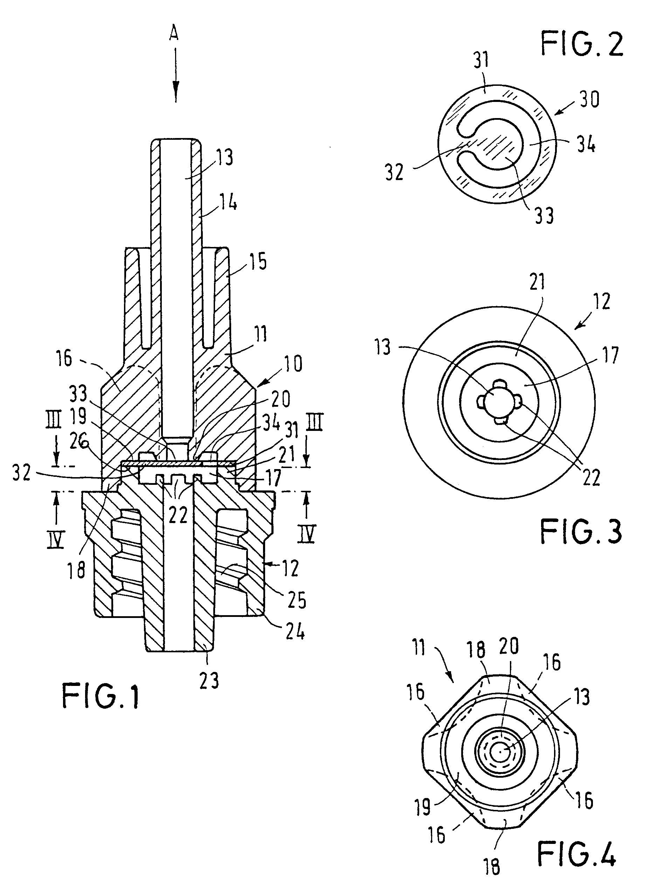

- Fig. 1: einen Längsschnitt durch das Rückschlagventil,
- Fig. 2: eine Draufsicht auf den Ventilkörper,
- Fig. 3: eine Draufsicht in Richtung der Linie III - III in Fig. 1 und
- Fig. 4: eine Draufsicht in Richtung der Linie IV - IV in Fig. 1.

Das Rückschlagventil besteht im wesentlichen aus einem rohrförmigen Gehäuse 10 aus Kunststoff und einem scheibenförmigen Ventilkörper 30 aus elastischem Material, insbesondere Naturkautschuk.

Das Gehäuse 10 ist aus zwei Teilen zusammengesetzt, die gemeinsam einen koaxialen geraden Durchlaß 13 bilden. Ein oberes Teil dient als Schlauchansatz 11. Ein Außenkonus 14 ist mit radialem Abstand von einem Mantelteil 15 umgeben, das das Ende eines auf den Außenkonus 14 aufgesteckten Schlauches klemmend aufnimmt. Der nicht gezeichnete Schlauch kommt von einem Infusionsgerät und ist von Infusionsflüssigkeit in Richtung des Pfeils A durchströmt. Am äußeren Umfangsbereich des Schlauchansatzes 11 befinden sich Griffmulden 16, welche zur besseren Handhabung beim An- bzw. Abkoppeln des Gehäuses 10 an eine weiterführende Leitung benötigt werden. Im unteren, stirnseitigen Bereich des Schlauchansatzes 11 befindet sich eine kreisförmige Aussparung 17, die von einem als Griffplatte ausgebildeten Randteil 18 umgeben ist. Im inneren Fußbereich des polygonalen Randteiles 18 ist eine kreisförmige Ringfläche 19 ausgebildet, die nach außen abfallend abgeschrägt ist. Die Ringfläche 19 ist nachfolgend als "obere" Ringfläche bezeichnet; sie umgibt konzentrisch einen ringförmigen Ventilsitz 20 mit ebener Sitzfläche, die in der Ebene der tiefsten äußeren Stelle der Ringfläche 19 liegt.

Das untere Teil des Gehäuses 10 ist ein Ansatz mit verriegelbarer Kegelverbindung mit Außenkegel 12. Auf der oberen Seite des Ansatzes 12 ist ein axial gerichteter Ringbund 21 vorgesehen, dessen Außendurchmesser dem Innendurchmesser des Randteils 18 des Schlauchansatzes 11 angepaßt ist und der in diesen Randteil 18 einsteckbar und durch US-Verschweißung oder Verklebung unlösbar mit diesem verbindbar ist. Die Stirnfläche des Ringbundes 21 bildet eine "untere" Ringfläche 26, die mit der gleichen Schräge wie die obere Ringfläche 19 versehen ist. Die beiden einander gegenüberliegenden Ringflächen 19 und 26 bilden einen Spalt, dessen Bestimmung nachfolgend erläutert wird.

Der Ringbund 21 umgibt einen Kranz von säulenförmigen axialen Nasen 22, die sich unmittelbar an den Rand des Durchlasses 13 anschließen und unter dem Ventilsitz 20 vorgesehen sind. Ein Außenkonus 23 des Ansatzes 12 ist mit radialem Abstand von einem Mantelteil 24 umgeben, das auf seiner Innenfläche ein Gewinde 25 aufweist, das zum Ankoppeln eines Konnektors einer Hahnbank, Kanüle oder anderer Systeme dient, die in Verbindung mit Infusionspumpe und deren Leitungen verwendet werden können.

Der Ventilkörper 30 besteht aus kreisförmig zugeschnittenem Flachmaterial, in dem zwei konzentrische Elemente ausgebildet sind. Ein äußerer kreisförmiger Dichtring 31 hat eine radiale Breite, die der radialen Breite der beiden zusammenpassenden oberen und unteren Ringflächen 19 und 26 im wesentlichen entspricht. Der Dichtring 31 bildet einen Ventilkörperring mit permanenter Dichtfunktion. Vom Innenumfang des Dichtringes 31 geht ein schmaler radialer Steg 32 nach innen aus, der eine Ventilscheibe 33 trägt. Die Ventilscheibe 33 hat Kreisform und ist mit kreisringförmigem Abstand 34 konzentrisch innerhalb des Dichtringes 31 angeordnet. Ihre Aufhängung an dem radialen Steg 32 ermöglicht eine Auslenkung quer zur Ebene des Flachmaterials. Der Durchmesser der Ventilscheibe 33 ist größer als der Durchmesser des Durchlasses 13 des Gehäuses 10, vorzugsweise größer als der Außendurchmesser des Ventilsitzes 20. Der Dichtring 31 wird zwischen den beiden Ringflächen 19 und 26, deren gegenseitiger Abstand im montierten Zustand des Schlauchansatzes 11 und des Ansatzes 12 etwa gleich der Dicke des Dichtringes 31 ist, eingespannt. Da infolge der Abschrägung der Ringflächen 19 und 26 der Dichtring 31 entsprechend schräg angestellt ist, wird der Dichtscheibe 33 eine Vorspannung erteilt, die sie in Schließstellung gegen den Ventilsitz 20 drückt. Der höchste Punkt des Ventilsitzes 20 liegt in der Ebene des tiefsten Punktes der Ringfläche 19. Die säulenförmigen Nasen 22, die mit Abstand unterhalb der Ventilscheibe 33 enden, sollen verhindern, daß die Ventilscheibe 33 infolge des Drucks der in Richtung des Pfeils A durch die Vorrichtung fließenden Infusionslösung den stromabwärts befindlichen Teil des Durchlasses 13 verschließen kann.

Im Normalfall fließt die von Infusionsbehältnissen kommende Infusionslösung in den Schlauchansatz 11 hinein und öffnet bei einer Wassersäule von ca. 10 cm die Ventilscheibe 33, indem sie sie von dem Ventilsitz 20 nach unten wegdrückt. Da die Ventilscheibe 33 gegen die Kuppen der Nasen 22 zur Anlage kommt, kann die Infusionslösung durch die Zwischenräume zwischen den Nasen 22 in den unteren Teil des Durchlasses 13 einströmen. Sind in einem Mehrfachinfusionsleitungssystem sowohl Schwerkraftleitung(en) als auch energetisch unterstützte Pumpenleitung(en) kombiniert, kann durch eine Leitungsstenose, z. B. eine im Patienten liegende, verschlossene Kanüle, die Infusionslösung aus der Pumpenleitung(en) in Richtung der Schwerkraftleitung(en) und das damit verbundene Infusionslösungsbehältnis(se) fließen. Eine nicht kontrollierte Patientenversorgung ist damit vorgegeben.

Sobald der Fließdruck der von der Infusionspumpe kommenden Infusionslösung höher ist als der Fließdruck der von einem Infusionsbehältnis über ein Schwerkraftinfusionsgerät ankommenden, lediglich der Schwerkraft unterworfenen Infusionslösung, wird die Ventilscheibe 33 gegen den Ventilsitz 20 gedrückt und schließt den Durchlaß 13 dicht ab. Die Funktionsfähigkeit des Ventils und die unidirektionale Abdichtfähigkeit des Ventilkörpers 30 muß vorzugsweise und vor allem auch bei einem von der Pumpe erzeugten Schließfluß im Bereich von nur 1 ml/h geförderter Infusionslösung(en) gegeben sein. Es wird so zuverlässig verhindert, daß die mit der Infusionspumpe(n) geförderte Infusionsflüssigkeit(en) in den Leitungsweg(e) des Schwerkraftinfusionsgerätes(e) gelangt. Sobald der Fließdruck der von der Infusionspumpe(n) geförderten Infusionslösung(en) höher ist als der Fließdruck der aus dem Schwerkraftinfusionsgerät(en) kommenden Infusionsflüssigkeit(en), wird die Ventilscheibe 33 gegen den Ventilsitz 20 des Schlauchansatzes 11 gedrückt, wobei ihre Vorspannung zu unmittelbarer, weitgehend trägheitsloser Funktion beiträgt, und sie sperrt den Durchlaß 13 dicht ab. Damit kann keine Infusionslösung(en) aus der durch die Infusionspumpe(n) betriebenen Flüssigkeitsleitung(en) in die lediglich unter Schwerkrafteinwirkung befindliche Infusionsleitung(en) einschließlich des zugeordneten Infusionslösungsbehältnisses(e) gelangen.

## Patentansprüche

1. Rückschlagventil mit einem Gehäuse (10) mit axialem Durchlaß (13) und einem Ventilsitz, mit dem eine Ventilscheibe (33) aus elastischem Material zusammenwirkt, die innerhalb eines Dichtringes (31) angeordnet und über eine örtliche Anschlußstelle (32) mit dem Dichtring (31) verbunden ist, wobei der Dichtring (31) zwischen einer oberen und einer unteren Ringfläche (19, 26) des Gehäuses (10), die den Ventilsitz (20) konzentrisch umgeben, eingespannt ist
und das Gehäuse (10) in eine Flüssigkeitsleitung eines Infusions- oder Transfusionsgerätes eingesetzt ist und der Durchlaß (13) Flüssigkeit führt.

2. Rückschlagventil nach Anspruch 1, dadurch gekennzeichnet, daß die zusammenpassenden oberen und unteren Ringflächen (19, 26) des Gehäuses (10) nach außen abfallend abgeschrägt sind, um den Dichtring (31) derart einzuspannen, daß die Ventilscheibe (33) in Schließstellung vorgespannt ist,
und daß eine Stirnfläche des ringförmigen Ventilsitzes (20) in der Ebene der tiefsten Stelle der oberen Ringfläche (19) des Gehäuses (10) liegt.

3. Rückschlagventil nach Anspruch 1 oder 2, dadurch gekennzeichnet, daß unterhalb und innerhalb der unteren Ringfläche (26) mindestens eine axiale Nase (22) angeordnet ist, die als Anschlag für die Ventilscheibe (33) dient.

4. Rückschlagventil nach Anspruch 3, dadurch gekennzeichnet, daß ein Kranz von Nasen (22) vorgesehen ist, die den Durchlaß (13) umgeben.

5. Rückschlagventil nach einem der Ansprüche 1 bis 4, dadurch gekennzeichnet, daß die Ventilscheibe (33) und der Dichtring (31) aus Flachmaterial einstückig ausgebildet sind, und daß die Anschlußstelle als radialer Steg (32) gestaltet ist.

6. Rückschlagventil nach Anspruch 5, dadurch gekennzeichnet, daß zwischem dem Dichtring (31) und der Ventilscheibe (33) ein der Länge des Stegs (32) entsprechender umfangsmäßiger Zwischenraum (34) vorhanden ist.

7. Rückschlagventil nach Anspruch 5 oder 6, dadurch gekennzeichnet, daß die beiden Seitenränder des Stegs (32) einwärts gebogen sind.

8. Rückschlagventil nach Anspruch 5 oder 6, dadurch gekennzeichnet, daß die beiden Seitenränder des Stegs gegen den Dichtring (31) konvergieren.

9. Rückschlagventil nach Anspruch 5, dadurch gekennzeichnet, daß das Flachmaterial der Ventilscheibe (33) und des Dichtringes (31) Naturkautschuk, Silikonkautschuk oder ein thermoplastisches Elastomer ist.

10. Rückschlagventil nach einem der Ansprüche 1 bis 9, dadurch gekennzeichnet, daß das rohrförmige Gehäuse (10) zweiteilig aus einem Schlauchansatz (11) mit dem Ventilsitz (20) und der oberen Ringfläche (19) und aus einem Konnektor-Ansatz (12) mit der unteren Ringfläche (26) gebildet ist, die einen Spalt zur klemmenden Aufnahme des Dichtringes (31) definieren.
